# EUROPÄISCHE PATENTSCHRIFT

(11) **EP 0 699 671 B1**
(45) Veröffentlichungstag und Bekanntmachung des Hinweises auf die Patenterteilung: **23.12.1998**
(21) Anmeldenummer: 95810517.3
(22) Anmeldetag: 16.08.1995
(51) Int. Cl.: C07D 333/38

(54) **Verfahren zur Herstellung von Thiophen-2,5-dicarbonsäure und deren Dichlorid**
Process for the preparation of thiophene-2,5-dicarboxylic acid and of its dichloride
Procédé pour la préparation de l'acide thiophène-2,5-dicarboxylique et de son dichlorure

(30) Priorität: 25.08.1994 CH 2607/94
(43) Veröffentlichungstag der Anmeldung: 06.03.1996
(73) Patentinhaber: Ciba Specialty Chemicals Holding Inc., 4057 Basel (CH)
(72) Erfinder: Orban, Ivan, Dr., CH-4052 Basel (CH)

(56) Entgegenhaltungen:
- CH-A- 426 870
- DD-A- 129 448
- DE-B- 1 210 888

## Beschreibung

Die Erfindung betrifft ein Verfahren zur Herstellung von Thiophen-2,5-dicarbonsäure und deren Dichlorid.

Thiophen-2,5-dicarbonsäure dient beispielsweise als Zwischenprodukt bei der Herstellung von optischen Aufhellern. Dazu muß sie in einer bestimmten Reinheit vorliegen. Es sind eine Reihe von Verfahren zu ihrer Herstellung bekannt, die über das Dichlorid dieser Säure als Zwischenprodukt verlaufen.

Ein Beispiel für solch ein Verfahren ist aus der Patentschrift DD-B-129 448 bekannt: Thionylchlorid und Adipinsäure sowie Pyridin als Katalysator werden zusammen erhitzt. Man kommt so zum Säurechlorid, das mit Natronlauge hydrolysiert wird, worauf die Säure mit Mineralsäure ausgefällt wird.

Es wurde nun gefunden, daß das Verfahren entscheidend verbessert werden kann:

Die Erfindung betrifft somit ein Verfahren zur Herstellung von Thiophen-2,5-dicarbonsäure-dichlorid durch Umsetzung von Thionylchlorid und Adipinsäure mit Pyridin als Katalysator, dadurch gekennzeichnet, daß
**A)** ein Teil Adipinsäure zu 3 bis 6 Teilen Thionylchlorid, vermischt mit einer katalytischen Menge Pyridin, zudosiert wird,
**B)** daß weitere 4 bis 7 Teile Thionylchlorid bei einer Temperatur im Bereich von 85 bis 95°C zugegeben werden,
**C)** überschüssiges Thionylchlorid und flüchtige Nebenprodukte bei vermindertem Druck entfernt werden, und
**D)** die Reaktion bei 140 bis 160°C zu Ende geführt wird. Gegebenenfalls kann man
**E)** aus dem entstandenen Chlorid durch Hydrolyse mit wäßriger Erdalkali- oder Alkalihydroxidlösung ein Salz der Thiophen-2,5-dicarbonsäure und
**F)** aus diesem mit einer Mineralsäure die freie Säure herstellen. Somit betrifft die Erfindung auch ein Verfahren zur Herstellung von Thiophen-2,5-dicarbonsäure und deren Salzen.

Zweckmäßig wird bei Schritt **A** 1 Teil Adipinsäure zu der 4-bis 5-fachen Menge Thionylchlorid zudosiert, und in Schritt B werden weitere 5 bis 6 Teile Thionylchlorid, bezogen auf Adipinsäure, zudosiert.

Pyridin ist bei Schritt **A** in katalytischen Mengen vorhanden. Bevorzugt sind 0,05 bis 0,2 mol Pyridin pro mol Adipinsäure anwesend, besonders bevorzugt 0,08 bis 0,12 mol.

Die Temperatur beträgt bei Schritt **A** zweckmäßig 20 bis 90°C, bevorzugt 30 bis 60°C, bei Schritt **B** liegt sie bevorzugt bei 87 bis 93°C.

Bei dem Destillationsschritt **C** wird zweckmäßig zunächst bis zu einem Druck von ca. 200 mbar das überschüssige Thionylchlorid abdestilliert und dann der Druck weiter auf 50 bis 10, bevorzugt 40 bis 20 mbar vermindert, wobei die Temperatur jeweils z.B. 80 bis 110°C, vorzugsweise 90 bis 105°C und besonders bevorzugt 90 bis 100°C beträgt.

Nach der Destillation wird der Unterdruck zweckmäßig beseitigt und vorzugsweise bei Normaldruck weitergearbeitet. Schritt **D** wird zweckmäßig bei 140 bis 160°C, bevorzugt bei einer Temperatur um 150°C durchgeführt. Das erhaltene Säurechlorid kann in üblicher Weise isoliert werden, z.B. durch physikalische Methoden wie Chromatographie oder Destillation. Vorzugsweise wird es bei vermindertem Druck abdestilliert.

Bei dem Verseifungsschritt **E** wird zweckmäßig das Säurechlorid in wäßrige Lauge (Alkali- oder Erdalkalihydroxidlösung) eingerührt. Geeignet dafür sind beispielsweise Natron- oder Kalilauge, besonders ca. 8 bis 15%ige Natronlauge.

Durch Ansäuern mit Mineralsäuren (Schritt **F**) wird aus dem entstanden Salz die freie Säure hergestellt. Geeignet hierfür sind starke Mineralsäuren wie Salz-, Schwefel- oder Phosphorsäure, vor allem Schwefelsäure. In einer bevorzugten Ausführungsform wird zunächst leicht auf pH 2 bis 5,5 , vorzugsweise 4 bis 5,2, angesäuert und Trübstoffe abfiltriert, bevor weitere Säure zugegeben wird, worauf das Salz aus der Lösung ausfällt.

Das hier beschriebene neue Verfahren ist für eine industrielle Anwendung aus folgenden Gründen wesentlich besser geeignet als das aus DD-B-129 448 bekannte Verfahren:
■ Die Reaktionszeiten sind bei dem erfindungsgemäßen Verfahren erheblich kürzer. Wenn man beispielsweise das Thionylchloridwie beschrieben etwa 20 Stunden auf die Adipinsäure einwirken läßt (Schritte **A**, **B**), erreicht man bereits hervorragende Ausbeuten, während die entsprechende Zeit im im Ausführungsbeispiel von DD-B-129 448 67 Stunden beträgt. Noch ausgeprägter ist der Unterschied beim Destillationschritt **C**: Die langsame Destillation von ca. 25 Stunden ist ein ein essentieller Teil des Verfahrens von DD-B-129 448, währendman beim vorliegenden Verfahren mit einer Destillationszeit von beispielsweise ein bis zwei Stunden auskommt.
■ Die Volumen-Ausbeute ist bei den Schritten **A** bis **D** gegenüber dem Verfahren aus DD-B-129 448 verdoppelt worden: Bei einem Mol Adipinsäure beträgt das anfängliche maximale Volumen (Wegen der Zersetzung des Thionylchlorids steigt das Volumen im weiteren Verlauf nicht weiter an.) 440 ml, während es im Ausführungsbeispiel von DD-B-129 448 880 ml sind.
■ Trotz kürzeren Reaktionszeiten und über den Gesamtzeitraum gesehen im Durchschnitt tieferen Temperaturen ist die erzielbare Ausbeute mit 55% d.Th. überraschenderweise höher als bei dem Verfahren aus DD-B-129 448 (49%).

Mit der Bereitstellung des vorliegenden Verfahrens eröffnen sich aus den obigen Gründen wesentlich verbesserte Möglichkeiten, die Synthese der Thiophen-2,5-dicarbonsäure in großem Maßstab industriell zu realisieren.

Die nach dem erfindungsgemäßen Verfahren hergestellten Verbindungen werden beispielsweise als Zwischenprodukte bei der Herstellung von optischen Aufhellern verwandt. Es lassen sich z.B. durch Umsetzung der Dicarbonsäure mit ortho-Aminophenolen optische Aufheller und Fluoreszenzfarbstoffe aus der Reihe der Bis-Benzoxazole herstellen (S.z.B. US-A-3,127,416).

Aus dem nach Schritt **D** vorliegenden Dicarbonsäurechlorid läßt sich auch direkt die Thiophen-2,5-dicarbonsäure herstellen, wenn zwei Moleküle HCl abgespalten werden. Dies kann z.B. durch Erwärmen auf 90 bis 100°C geschehen, wie in CH-B-426 870 beschrieben. Geschieht dies in Anwesenheit geeigneter Reaktionspartner wie der oben erwähnten ortho-Aminophenole, so können direkt aus dem Dicarbonsäurechlorid optische Aufheller hergestellt werden.

Die Vorteile des erfindungsgemäßen Verfahrens kommen somit auch der direkten Herstellung der Dicarbonsäure aus dem Dicarbonsäurechlorid zugute. Bevorzugt ist hier die Ausführungsform, bei der als Zwischenprodukte (Schritt **E**) die Salze der Dicarbonsäure gebildet werden.

Die folgenden Beispiele erläutern die Erfindung weiter, ohne sie jedoch zu beschränken. Teile- und Prozentangaben beziehen sich - wie auch in der übrigen Beschreibung - sofern nicht anders angegeben, auf das Gewicht.

### Beispiel 1:

In einem Reaktionsgefäß mit Rührer, Thermometer, Kühler, Tropftrichter und Inertgasspülung werden 655 g Thionylchlorid (5,5 mol) und 11 g Pyridin (0,14 mol) vorgelegt und auf 30 -35°C erwärmt. Bei dieser Temperatur werden innerhalb 1 h über einen Feststoff-Dosiertrichter 204,4 g Adipinsäure (1,4 mol) zudosiert. Das entweichende Salzsäure/Schwefeldioxid-Gasgemisch wird über eine kleine Füllkörperkolonne und einen darauf montierten Rückflußkühler abgleitet und absorbiert.

Nun wird die Reaktionsmischung auf eine Rückflußtemperatur von 90°C geheizt, und anschließend werden innerhalb 20 h insgesamt 907 g Thionylchlorid (7,6 mol) so zudosiert, daß im Kolben bei konstanter Innentemperatur von 90°C während der ganzen Zeit ein guter Rückfluß herrscht. Daraufhin werden das überschüssige Thionylchlorid bei 90-100°C /1000 -200 mbar und das entstandene Schwefelmonochlorid bei 100°C/200-20 mbar abdestilliert. Der Druck wird mit Stickstoff entlastet und der Destillationsrückstand innerhalb 45 Minuten auf 150°C geheizt und 3 h bei dieser Temperatur gehalten.

Es werden 190 g Thiophen -2,5-dicarbonsäurechlorid als leicht gelbliches Öl bei 132-142°C/7-8 mbar abdestilliert. Die ca. 100°C heiße Schmelze des Dicarbonsäurechlorids (Smp. 45-47°C) wird unter Rühren bei 40-50°C zu einer Mischung von 1600 g Wasser und 490 g 30%iger Natronlauge portionsweise zudosiert. Die entstandene Lösung wird mit ca. 47 g Schwefelsäure auf pH 4,8 bis 5,0 eingestellt und anschließend mit 13 g Aktivkohle und 30 g Filtrierhilfsmittel (Kieselgur Clarcel Dif B) geklärt. Zum geklärten Filtrat werden bei 20 bis 25°C 89 g Schwefelsäure (93%) zugetropft. Die entstandenen feinen, hellbeigen Kristalle werden abfiltriert und mit Wasser gewaschen. Nach dem Trocknen erhält man 133 g Thiophen-2,5-dicarbonsäure (55% d. Th.). Titration und GC Bestimmung ergeben Reinheiten von 96-99%. Smp.: 324-330°C.

## Patentansprüche

1. Verfahren zur Herstellung von Thiophen-2,5-dicarbonsäure-dichlorid durch Umsetzung von Thionylchlorid und Adipinsäure mit Pyridin als Katalysator, dadurch gekennzeichnet, daß
**A)** ein Teil Adipinsäure zu 3 bis 6 Teilen Thionylchlorid, vermischt mit einer katalytischen Menge Pyridin, zudosiert wird,
**B**) daß weitere 4 bis 7 Teile Thionylchlorid bei einer Temperatur im Bereich von 85 bis 95°C zugegeben werden,
**C)** überschüssiges Thionylchlorid und flüchtige Nebenprodukte bei vermindertem Druck entfernt werden, und
**D)** die Reaktion bei 140 bis 160°C zu Ende geführt wird.

2. Verfahren nach Anspruch **1**, dadurch gekennzeichnet, daß nach den Schritten **A** bis **D**
**E)** aus dem entstandenen Chlorid durch Hydrolyse mit wäßriger Erdalkali- oder Alkalihydroxidlösung ein Salz der Thiophen-2,5-dicarbonsäure hergestellt wird.

3. Verfahren nach Anspruch **2**, dadurch gekennzeichnet, daß nach den Schritten **A** bis **E**
**F**) aus dem Salz der Thiophen-2,5-dicarbonsäure durch Umsetzung mit einer Mineralsäure die freie Säure hergestellt wird.

4. Verfahren nach Anspruch **1**, dadurch gekennzeichnet, daß bei Schritt A 0,05 bis 0,2 mol Pyridin pro mol Adipinsäure anwesend sind.

5. Verfahren nach Anspruch **1**, dadurch gekennzeichnet, daß die Temperatur bei Schritt **A** 20 bis 90°C beträgt.

6. Verfahren nach Anspruch **1**, dadurch gekennzeichnet, daß die Temperatur bei Schritt **B** 87 bis 93°C beträgt.

7. Verfahren nach Anspruch **1**, dadurch gekennzeichnet, daß der Destillationsschritt **C** bei einem Druck von 10 -50 mbar und einer Temperatur von 80 bis 110°C durchgeführt wird.

8. Verfahren nach Anspruch **1**, dadurch gekennzeichnet, daß die Reaktionszeit für die Schritte **A** bis einschließlich **D** bei einem 1-2-molaren Ansatz unter 30 h liegt.

## Claims

1. A process for the preparation of the dichloride of thiophene-2,5-dicarboxylic acid by reacting thionyl chloride and adipic acid with pyridine as catalyst, which process comprises
**A**) adding one part of adipic acid to 3 to 6 parts of thionyl chloride, mixed with a catalytic amount of pyridine,
**B**) adding a further 4 to 7 parts of thionyl chloride at a temperature in the range from 85 to 95°C,
**C**) removing excess thionyl chloride and volatile byproducts under reduced pressure, and
**D**) bringing the reaction to completion at from 140 to 160°C.

2. A process according to claim **1**, which comprises preparing after steps **A** to **D**
**E**) a salt of thiophene-2,5-dicarboxylic acid by hydrolysing the resulting chloride with an aqueous solution of an alkaline earth metal hydroxide or of an alkali metal hydroxide.

3. A process according to claim **2**, which comprises preparing after steps **A** to **E**
**F**) the free acid from the salt of thiophene-2,5-dicarboxylic acid by reaction with a mineral acid.

4. A process according to claim **1**, wherein there is in step **A** from 0.05 to 0.2 mol of pyridine per mole of adipic acid.

5. A process according to claim **1**, wherein the temperature in step **A** is from 20 to 90°C.

6. A process according to claim **1**, wherein the temperature in step **B** is from 87 to 93°C.

7. A process according to claim **1**, wherein distillation step **C** is carried out under a pressure of 10-50 mbar and at a temperature from 80 to 110°C.

8. A process according to claim **1**, wherein the reaction time for steps **A** up to and including **D** is below 30 h in a 1-2-molar batch.

## Revendications

1. Procédé de préparation d'acide thiophène-2,5-dicarboxylique par réaction de chlorure de thionyle et d'acide adipique, en présence de la pyridine comme catalyseur, caractérisé en ce que
A) on ajoute une partie d'acide adipique à 3 à 6 parties de chlorure de thionyle, mélangé aux quantités catalytiques de pyridine comme catalyseur,
B) on ajoute les autres 4 à 7 parties de chlorure de thionyle à une température comprise entre 85 et 95 °C,
C) on élimine l'excès en chlorure de thionyle et les sous-produits volatils sous pression réduite, et
D) on met en oeuvre la réaction à une température de 140 à 160 °C jusqu'à sa fin.

2. Procédé selon la revendication 1, caractérisé en ce que, après les étapes A à D, on prépare
E) à partir du chlorure obtenu par hydrolyse avec une solution aqueuse d'hydroxyde de métaux alcalino-terreux ou alcalins, un sel d'acide thiophène-2,5-dicarboxylique.

3. Procédé selon la revendication 2, caractérisé en ce que après les étapes A à E, on prépare
F l'acide libre à partir du sel d'acide thiophène-2,5-dicarboxylique avec un acide minéral.

4. Procédé selon la revendication 1, caractérisé en ce que dans l'étape A, 0,05 à 0,2 mole de pyridine sont présents par mole d'acide adipique.

5. Procédé selon la revendication 1, caractérisé en ce que la température à l'étape A est de 20 à 90 °C.

6. Procédé selon la revendication 1, caractérisé en ce que la température à l'étape B est de 87 à 93 °C.

7. Procédé selon la revendication 1, caractérisé en ce que l'on met en oeuvre l'étape de distillation C sous une pression de 10 à 50 mbar et à une température de 80 à 110 °C.

8. Procédé selon la revendication 1, caractérisé en ce que la durée réactionnelle des étapes A à D compris, pour une charge de 1 à 2 moles, est inférieure à 30 heures.
